(19) Europäisches Patentamt
European Patent Office
Office européen des brevets

(11) Veröffentlichungsnummer: **0 308 786**
**A1**

(12) **EUROPÄISCHE PATENTANMELDUNG**

(21) Anmeldenummer: **88114980.1**

(51) Int. Cl.⁴: **A61L 2/18**

(22) Anmeldetag: **14.09.88**

(30) Priorität: **25.09.87 DE 3732379**

(43) Veröffentlichungstag der Anmeldung:
**29.03.89 Patentblatt 89/13**

(84) Benannte Vertragsstaaten:
**AT BE CH DE ES FR GB GR IT LI LU NL SE**

(71) Anmelder: **BAYER AG**
**D-5090 Leverkusen(DE)**

(72) Erfinder: **Schmitt, Hans-Georg, Dr.**
**Am Oberend 13**
**D-4150 Krefeld 1(DE)**
Erfinder: **Herbst, Josef**
**Breslauer Strasse 27**
**D-4150 Krefeld 11(DE)**
Erfinder: **Hoffmann, Manfred**
**Ingerstrasse 24**
**D-4154 Tönisvorst 1(DE)**
Erfinder: **Heiss, Rüdiger, Dr.**
**Heide 60**
**D-4155 Greftrath 1(DE)**
Erfinder: **Wegner, Gerd**
**Steiermarkstrasse 75**
**D-4100 Duisburg 28(DE)**

(54) **Desinfektion von Abformungen.**

(57) Mikrobizide Phenolverbindungen werden zur Desinfektion von Abformungen, insbesondere für zahntechnische Arbeiten, verwendet.

EP 0 308 786 A1

Xerox Copy Centre

## Desinfektion von Abformungen

Die Erfindung betrifft die Verwendung von mikrobiziden Phenolverbindungen zur Herstellung von Desinfektionsmitteln, die für zahntechnische Abformungen geeignet sind.

In der zahnärztlichen Praxis werden Abdrücke genommen, um auf den daraus gewonnenen Modellen zahntechnische Arbeiten anzufertigen.

Als Abformmaterialien werden vorwiegend Hydrokolloide z.B. Alginate, und Elastomere (Silikone, Polyether und Polysulfide) verwendet. Bevorzugt kommen Alginate bei der Herstellung von herausnehmbaren Prothesen und kieferorthopädischen Geräten zum Einsatz, während Elastomere für die Anfertigung von Inlays, Kronen und Brücken Anwendung finden.

Es ist bekannt, daß Mikroorganismen der Mundhöhle durch zahnärztliche Abformungen auf die daraus erstellten Gipsmodelle übertragen werden können (Journal of Prosthetic Dentistry 49, 210 bis 211 (1983)).

In Anbetracht des Infektionsrisikos für den behandelnden Zahnarzt sowie das Personal der Praxis und des Dentallabors, ist der Desinfektion von Abformungen besondere Bedeutung beizumessen.

Während für die Desinfektion von Instrumenten, Geräten und Mobiliar der zahnärztlichen Praxis Hygienevorschriften existieren (beispielsweise die Unfallverhütungsvorschrift der Berufsgenossenschaft für Gesundheitsdienst und Wohlfahrtspflege (1982)) und die entsprechenden Desinfektionsmittel empfohlen werden (beispielsweise die Liste der deutschen Gesellschaft für Hygiene und Mikrobiologie (1981)), bleiben in bezug auf die Desinfektion von Abformungen Unsicherheiten für den Zahnarzt bestehen. Die für die Instrumenten- und Flächendesinfektion eingesetzten Präparate können in den empfohlenen Anwendungskonzentrationen und Anwendungszeiten nicht ohne weiteres für die Desinfektion von Abformungen angewendet werden, da Angaben zur Materialverträglichkeit und ausreichenden bakteriologischen Wirksamkeit auf der Oberfläche der jeweiligen Abformung fehlen.

Desinfektionsmittel für Abformungen sollen folgenden Anforderungen entsprechen:

1. Die auf der Abdruckoberfläche vorhandenen Keimzahlen sollten ausreichend reduziert werden.
2. Dimensions- und Oberflächenveränderungen an der Abformung und den daraus resultierenden Modellen dürfen nicht auftreten.
3. Die Desinfektion soll in einer für den Praxisablauf akzeptablen Zeit erfolgen.
4. Die Desinfektionsmittel sollen leicht und gefahrlos anzuwenden und zu entsorgen sein.
5. Die Desinfektionsmittel sollen für alle Abformmaterialien einsetzbar sein.

In der Literatur sind zahlreiche Untersuchungen mit handelsüblichen Desinfektionsmitteln bekannt, die hinsichtlich Punkt 2. (Materialverträglichkeit) geprüft wurden, ohne die anderen Anforderungen zu berücksichtigen. In den wenigen Publikationen, die alle genannten Bedingungen (1.-5.) berücksichtigen (z.B. Dtsch zahnärztl. Z 742-748, 1983; Dtsch zahnärztl. Z 38, 234-237, 1983) erfüllt keines der geprüften Präparate alle Anforderungen. So erwies sich das auf Silikonabformungen getestete Benzoesäurepräparat Sagrodent® als nicht ausreichend bakterizid, während die geprüften Peressigsäurepräparate in ihrer bakteriologischen Wirkung nur auf Alginatabformungen untersucht wurden und zudem nur unter Einhaltung strikter Vorsichtsmaßnahmen anzuwenden sind.

Die erfindungsgemäße Formulierung gewährleistet bei praxisrelevanten Anwendungszeiten eine ausreichend hohe Reduktion pathogener Keime auf kontaminierten Abformungen, ohne daß die Abformgenauigkeit beeinträchtigt wird. Die Formulierung läßt sich universell und für den Anwender einfach und gefahrlos anwenden.

Durch Anwendung der erfindungsgemäßen Formulierung kann die Kontaminationskette Patient-Zahnarzt-Helferin-Zahntechniker frühzeitiger und damit wirkungsvoller unterbrochen werden als mit anderen z.B. von Weiß (Dissertation Köln 1984) beschriebenen Verfahren zur Desinfektion von Gipsmodellen, bei deren Herstellung dem Anmischwasser Chloramin zugefügt werden soll.

Mikrobizide Phenolverbindungen zur Flächen- oder Instrumentendesinfektion sind an sich bekannt (Ullmann-Enzyklopädie der technischen Chemie, 4. Auflage, 1975 Band 10, S. 41-58). Es handelt sich in der Regel um handelsübliche Mikrobizide, die nicht zue Desinfektion von Abformmaterialien entwickelt wurden.

Es seien beispielsweise mikrobizide Phenole der Formel

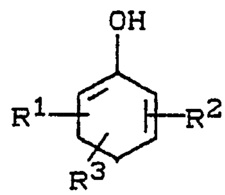

in der
$R^1$ und $R^2$ gleich oder verschieden sind und Wasserstoff, Chlor, Methyl, Benzyl oder Phenyl bedeuten und $R^3$ Wasserstoff oder Chlor bedeutet.

Als mikrobizide Phenolverbindungen seien beispielsweise 2-Phenylphenol, 2-Benzyl-4-chlorphenol, 4-Chlor-3-kresol und 4-Chlor-3,5-xylenol genannt. Bevorzugte mikrobizide Phenolverbindungen sind 2-Phenylphenol und 2-Benzyl-4-chlorphenol.

Es ist selbstverständlich möglich, Gemische der einzelnen mikrobiziden Phenolverbindungen einzusetzen.

Die erfindungsgemäßen Zubereitungen für die Anwendung enthalten im allgemeinen 2 bis 40 Gew.-%, bevorzugt 4 bis 12 Gew.-%, der mikrobiziden Phenolverbindung.

Die erfindungsgemäßen mikrobiziden Phenolverbindungen werden im allgemeinen in wäßriger Zubereitung zur Desinfektion der Abformungen verwendet.

Die erfindungsgemäßen Zubereitungen können gegebenenfalls noch Tenside, Lösemittel und/oder Komplexbildner enthalten.

Lösemittel sind im allgemeinen mit Wasser mischbare Lösemittel, z.B. Alkohole wie Isopropanol.

Als Tenside werden im allgemeinen anionische Tenside, z. B. sekundäre Alkylsulfonate mit einer durchschnittlichen Kettenlänge von $C_{12}$ bis $C_{14}$ eingesetzt.

Als Tenside seien beispielsweise genannt:
- Natriumalkansulfonat, mittlere Kettenlänge C 13.
- Sekundäres Natriumalkansulfonat, mittlere Kettenlänge C 15.

Es ist außerdem möglich, daß in den erfindungsgemäßen Zubereitungen Komplexbildner vorhanden sind. Beispielsweise sei Ethylendiamin-N,N,N′,N′-tetraessigsäurenatriumsalz genannt.

Die erfindungsgemäßen Desinfektionsmittelzubereitungen besitzen in der Regel einen pH-Wert im Bereich von 4 bis 12, bevorzugt von 6 bis 9. Gegebenenfalls kann ein entsprechender pH-Wert mit Hilfe von Milchsäure oder Natronlauge eingestellt werden.

Die erfindungsgemäßen Zubereitungen können beispielsweise die folgenden Zusammensetzungen haben:
2 bis 40 Gew.-Teile, bevorzugt 4 bis 12 Gew.-Teile der mikrobiziden Phenolverbindung,
10 bis 87 Gew.-Teile, bevorzugt 10 bis 60 Gew.-Teile Wasser,
10 bis 40 Gew.-Teile, bevorzugt 20 bis 30 Gew.-Teile eines Tensids,
0 bis 50 Gew.-Teile, bevorzugt 10 bis 30 Gew.-Teile, eines Lösemittels und/oder 0 bis 10 Gew.-Teile, bevorzugt 1 bis 7 Gew.-Teile eines Komplexbildners.

Die erfindungsgemäßen Zubereitungen werden als Konzentrat oder als fertige Gebrauchsverdünnung in den Handel gebracht.

In der Regel werden die Abformungen sofort nach Entnahme aus dem Munde des Patienten in die Gebrauchsverdünnung getaucht, die 0,1 bis 60 Gew.-Teile, bevorzugt 5 bis 40 Gew.-Teile der erfindungsgemäßen Zubereitung enthält. Damit wird eine ausreichende Desinfektion in für die Praxis geeigneter Anwendungszeit erzielt.

Es ist selbstverständlich auch möglich, die erfindungsgemäße Zubereitung bzw. deren Gebrauchsverdünnung für eine Sprühdesinfektion mit und ohne Treibgas anzuwenden.

Die Herstellung der erfindungsgemäßen Zubereitungen erfolgt im allgemeinen durch Zusammengabe der Komponenten und gute Durchmischung.

## Beispiele

1. Zur Prüfung der mikrobiziden Wirksamkeit der erfindungsgemäßen Desinfektionsmittelkombination wurde ein Konzentrat der folgenden Zusammensetzung durch Vermischen der Einzelbestandteile hergestellt.

Beispiel 1

6,5 Gew.-Teile 2-Phenylphenol
3,2 Gew.-Teile 2-Benzyl-4-chlorphenol
20,0 Gew.-Teile Isopropanol
25,0 Gew.-Teile sek. Na-alkansulfonat
1,0 Gew.-Teil Ethylendiamin-N,N,N′,N′-tetraessigsäure-Na-Salz
0,3 Gew.-Teile Milchsäure
44,0 Gew.-Teile Wasser

2. Prüfung der Materialverträglichkeit

Aus der unter Beispiel 1 genannten Rezeptur wurden 5- und 10 %ige Gebrauchslösungen unter Verwendung von Wasser hergestellt. In diese Lösungen wurden Abformungen aus Alginat, kondensationsvernetztem Silikon und additionsvernetztem Silikon getaucht. Die Expositionszeiten betrugen für das Alginat 5 Minuten, für die Silikone 15 Minuten. Anschließend wurde nach Abspülen mit Wasser die Verträglichkeit der Abformmaterialien mit Modellgips analog ADA-Spezifikation Nr. 18 bzw. 19 geprüft.

Die Dimensionsstabilität der Siliconabformmaterialien wurde analog der ADA-Spezifikation Nr. 19, die der Alginatabformung in Anlehnung an die im Tandlaege-bladet 86, 626-631, 1982 publizierte Prüfmethode getestet. Die geprüften Abformmaterialien erwiesen sich nach Anwendung des Desinfektionsmittels als dimensionsstabil und gipsverträglich.

Prüfung der bakteriologischen Wirksamkeit

A. Qualitativer Suspensionsversuch nach dem in Hygiene und Medizin 7, 325-327 (1982) publizierten Verfahren.

Mit folgenden Verdünnungen des Konzentrats nach Beispiel 1 werden nach einer Kontaktzeit von 5 Minuten die in Tabelle 1 angegebenen Keime abgetötet.

Tabelle 1

| Prüfkeime | Anwendungskonzentration |
|---|---|
| Staphylococcus aureus ATCC 6538 | 0,2 % |
| Escherichia coli ATCC 11229 | 0,4 % |
| Pseudomonas aeruginosa ATCC 15442 | 0,4 % |

B. Bestimmung des Desinfektionswirkung auf Abformmaterialoberflächen

1. Prüfkeime:

Staphylococcus aureus ATCC 6538
Escherichia coli ATCC 11229
Pseudomonas aeruginosa ATCC 15442
Candida albicans ATCC 10575

2. Abformmaterial:

Alginoplast s.h.
Mischungsverhältnis: 23 g Pulver und 50 ml Wasser (Temp. 20° C)

Herstellung der Prüfkörper:

Unter Beachtung der Anmischzeit von 30 sec, der Verarbeitungszeit von 60 sec und einer Abbindezeit von weiteren 210 sec werden mit Hilfe von Greiner Rodac-Schalen zylinderförmige Prüfkörper hergestellt und Stücke von 3 cm Durchmesser und 0,5 cm Höhe ausgestanzt.

4. Desinfektionsmittelprüfung

Die Prüfkeime werden vorkultiviert und die Ausgangskeimzahl wird bestimmt. In die Keimlösung ($5 \times 10^8$ - $2 \times 10^9$ Keime/ml) werden nach Zugabe von 10 % Rinderserum als Eiweißbelastung die frisch hergestellten Prüfkörper 3 Minuten getaucht und in die Desinfektionsmittel überführt.

Nach 5 Minuten Einwirkzeit Entnahme der Prüfkörper, Überführung in eine Abspüllösung und Bestimmung der Restkeimzahl sowohl in der Abspüllösung als auch auf dem Prüfkörper.

Tabelle 2

| Desinfektion von Alginat-Abformmassen | | | | | | | | | | | | | | | | | |
|---|---|---|---|---|---|---|---|---|---|---|---|---|---|---|---|---|---|
| Prüfkeim: | Konz. Gew.-% | Staphylococcus aureus | | | | Escherichia coli | | | | Pseudomonas aeruginosa | | | | Candida albicans | | | |
| | | KBE pro 1 ml ASL | | | | KBE pro 1 ml ASL | | | | KBE pro 1 ml ASL | | | | KBE pro 1 ml ASL | | | |
| Kontrolle | 0 | $2{,}1\text{-}2{,}4 \times 10^4$ | | | | $1{,}8\text{-}2{,}0 \times 10^4$ | | | | $8 \times 10^4\text{-}1{,}1 \times 10^5$ | | | | $2{,}4\text{-}3{,}0 \times 10^2$ | | | |
| Desinfektionsmittel gem. Bsp. 1 | 5 | 9 | 79 | 0 | 3 | ~200 | 4 | 4 | 76 | 156 | 168 | ~180 | 104 | 0 | 0 | 0 | 0 |
| | | KBE pro Prüfkörper | | | | KBE pro Prüfkörper | | | | KBE pro Prüfkörper | | | | KBE pro Prüfkörper | | | |
| Kontrolle | 0 | >500 | | >500 | | >500 | | >500 | | >500 | | >500 | | 49 | | 60 | |
| Desinfektionsmittel gem. Bsp. 1 | 5 | ~100 | ~100 | 23 | 22 | 38 | 7 | 25 | 26 | ~180 | ~200 | ~190 | ~200 | 0 | 0 | 0 | 0 |
| Bemerkungen: KBE = kolonienbildende Einheiten<br>ASL = Abspüllösung | | | | | | | | | | | | | | | | | |

## Ansprüche

1. Verwendung von Zubereitungen enthaltend mikrobizide Phenolverbindungen zur Desinfektion von Abformungen.

2. Verwendung nach Anspruch 1, dadurch gekennzeichnet, daß mikrobizide Phenole der Formel

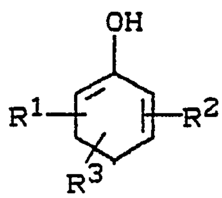

in der
$R^1$ und $R^2$ gleich oder verschieden sind und Wasserstoff, Chlor, Methyl, Benzyl oder Phenyl und
$R^3$ Wasserstoff oder Chlor bedeutet,
eingesetzt werden.

3. Verwendung nach den Ansprüchen 1 und 2, dadurch gekennzeichnet, daß als mikrobizide Phenolverbindungen 2-Phenyl-phenol und/oder 2-Benzyl-4-chlor-phenol eingesetzt werden.

4. Verwendung nach den Ansprüchen 1 bis 3, dadurch gekennzeichnet, daß die Zubereitungen 2 bis 40 Gew.-% der mikrobiziden Phenolverbindung enthalten.

5. Verwendung nach den Ansprüchen 1 bis 4, dadurch gekennzeichnet, daß die Zubereitungen Wasser and gegebenenfalls Tenside, Lösemittel und/oder Komplexbildner enthalten.

6. Verwendung nach Ansprüchen 1 bis 5, dadurch gekennzeichnet, daß die Gebrauchsverdünnung 0,1 bis 50 Gew.-% der Zubereitungen enthalten.

Europäisches Patentamt

# EUROPÄISCHER RECHERCHENBERICHT

Nummer der Anmeldung

EP 88 11 4980

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 105, Nr. 20, 17. November 1986, Seite 377, Ref.Nr. 178387f, Columbus, Ohio, US; S.P. HERRERA et al.: "Dimensional st of dental impressions after immersion disinfection", & J. AM. DENT. ASSOC. 1986, 113(3), 419-22 * Insgesamt * | 1 | A 61 L 2/18 |
| Y | IDEM | 2-6 | |
| Y | EP-A-0 147 223 (STERWIN AG) * Seite 3, Zeile 13 - Seite 4, Zeile 8 * | 2-6 | |
| A | FR-A-2 233 074 (W. HURKA) * Seite 5, Zeilen 6-9 * | 2-6 | |
| A | CHEMICAL ABSTRACTS, Band 104, Nr. 24, 16. Juni 1986, Seite 373, Ref.Nr. 213191r, Columbus, Ohio, US; B. BERGMAN et al.: "Alginate impression materials, dimensional stability and surface detail sharpness following treatment with disinfectant solutions", & SWED. DENT. J. 1985, 9(6), 255-62 * Insgesamt * | 1-6 | RECHERCHIERTE SACHGEBIETE (Int. Cl.4): A 61 L, A 61 K |

Der vorliegende Recherchenbericht wurde für alle Patentansprüche erstellt

| Recherchenort | Abschlußdatum der Recherche | Prüfer |
|---|---|---|
| DEN HAAG | 23-12-1988 | COUSINS-VAN STEEN G.I.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)